# EUROPEAN PATENT SPECIFICATION

(11) **EP 2 059 443 B1**
(45) Date of publication and mention of the grant of the patent: **03.11.2010**
(21) Application number: 07804857.6
(22) Date of filing: 29.08.2007
(51) Int. Cl.: B65B 3/00, A61M 5/178, A61M 5/00, G01T 7/02

(54) **AUTOMATIC MACHINE FOR FRACTIONATING A RADIOACTIVE LIQUID**
AUTOMATISCHE MASCHINE ZUR FRAKTIONIERUNG EINER RADIOAKTIVEN FLÜSSIGKEIT
MACHINE AUTOMATIQUE DE FRACTIONNEMENT D'UN LIQUIDE RADIOACTIF

(30) Priority: 30.08.2006 IT BO20060619
(43) Date of publication of application: 20.05.2009
(73) Proprietor: Tema Sinergie S.r.l., 48018 Faenza RA (IT)
(72) Inventor: BERTI, Mauro, 48018 Faenza (IT)
(74) Representative: Nesti, Antonio
(86) International application number: PCT/IB2007/002498
(87) International publication number: WO 2008/026050

(56) References cited:
- EP-A- 1 820 730
- WO-A-2004/108533
- WO-A-2005/002971
- WO-A-2007/110684
- FR-A- 2 739 565
- US-A- 5 479 969
- US-A- 5 911 252
- US-A1- 2005 278 066
- US-B1- 6 477 442

## Description

### Technical Field

The present invention relates to a machine and a method for fractionating a radioactive liquid into predetermined doses.

### Background Art

As is well known to experts in the sector, in the production and distribution of this type of liquids, due to the rapid decay of their radioactive activity, it is essential that as little time as possible elapses between the moment when production begins and the moment of delivery to the customer.

In general, to organise and optimise the entire process, the user is assigned a "booking", for a predetermined day and a precise time, for the delivery of a predetermined volume of liquid, fractionated into doses poured into a plurality of vials or syringes, having a predetermined radioactive activity.

There are also various prior art types of machines for fractionating these radioactive liquids which, to obtain the radioactive activity required by the customer, need an operator to manually enter all of the dispensing operating parameters before starting production. Such machine is disclosed for example in FR 2739565.

In practice, to programme the production of a predetermined volume of liquid, the operator prepares a spreadsheet, called a "recipe", with which, if one knows the formula for the decay of the radioactive activity and the time remaining until delivery, it is possible to set the parameters for automatic fractionation of the radioactive liquid in vials or syringes.

Fractionation is normally accompanied by labelling required by current legislation, the labels showing for example the value of the radioactivity, the date and the end customer reference.

However, these machines have several disadvantages.

A first disadvantage is due to the fact that these prior art machines do not integrate any automatic programming support for the fractionating process, based on the effective performance of the process.

Since the output of the production process prior to fractionation of the radioactive activity is not constant in terms of both total radioactive activity and specific radioactive activity and therefore is not predictable, correction of the dispensing operating parameters already entered is often necessary.

For this reason, if the values do not match, the "recipe" has to be reformulated to program the fractionating cycle again, with a consequent loss of time and therefore a greater decay in the activity of the liquid and a drop in its economic value.

Another constraint on the above-mentioned dispensing systems is caused by the maximum error margin legally allowed (+- 5%): vials or syringes outside the range are rejected and cannot be used.

Moreover, as previously described, since the time which elapses between production and delivery must be as short as possible, the manufacturer must ensure that the various operations are carried out at the maximum possible speed.

This reveals a second significant disadvantage, consisting of the fact that the imprecision of the value of radioactive activity obtained is generally caused, in prior art machines, by attempts to achieve the maximum dispensing speed using pumps which, for the entire dispensing period, operate at a compromise speed between the maximum value, to accelerate the transfer, and a low value, to obtain an acceptable level of precision.

### Disclosure of the Invention

Therefore, the present invention has for an aim to overcome the first disadvantage by providing an automatic machine for fractionating a radioactive liquid with an operatively integrated programming unit, according to claim 1 and a method according to claim 14.

The invention also provides a machine which in addition to the dispensing means uses at least one encoder-controlled peristaltic pump, which can be used to speed up the dispensing step without any loss of precision.

Accordingly, the machine for fractionating a radioactive liquid comprises a programming unit and a unit for controlling the production process, for controlling and if necessary correcting the fractionating cycle during the process.

### Brief Description of the Drawings

The technical features of the invention are clearly described in the claims below and its advantages are more apparent from the detailed description which follows, with reference to the accompanying drawings which illustrate a preferred, non-limiting embodiment of the invention, and in which:
Figure 1 illustrates a machine for fractionating a radioactive liquid in accordance with the present invention;
Figure 2 is a schematic view of the connections between the PLC unit and the respective remote data processing unit;
Figure 3 is a schematic view of the hydraulic circuit of the fractionating machine of the previous Figures;
Figure 4 illustrates a detail of a vial moving mechanism used in a machine in accordance with the present invention, and
Figure 5 illustrates the series of successive operating positions of a fractionating machine in accordance with the present invention.

### Detailed Description of the Preferred Embodiments of the Invention

In accordance with the accompanying drawings a machine 1 for fractionating a radioactive liquid is described.

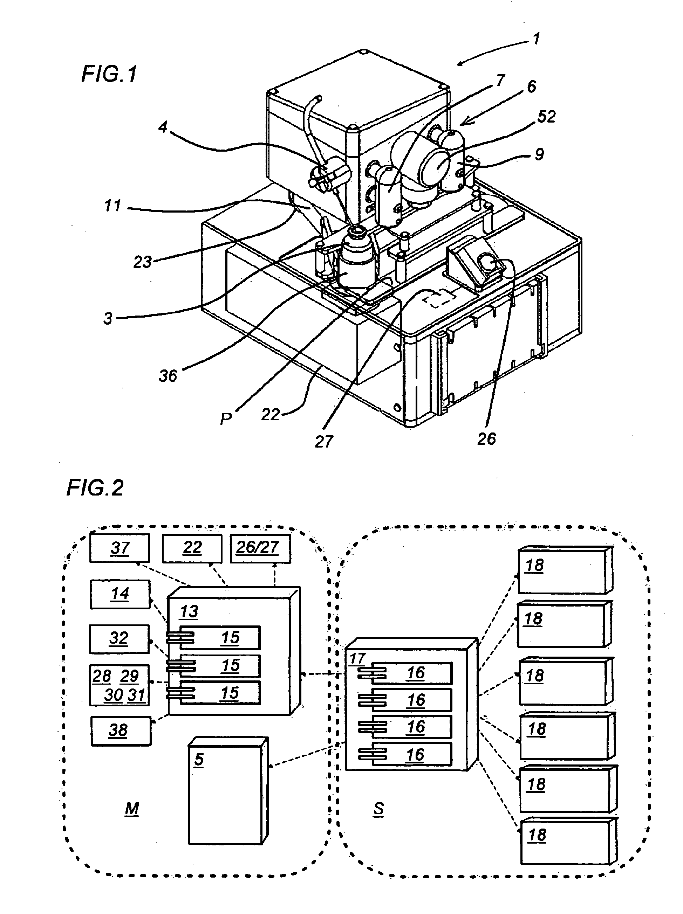

Figures 1, 3 and 5 illustrate a machine 1 for dispensing measured doses of a radioactive liquid in one or more vials 3 comprising:
- means 19 for receiving the radioactive liquid from a feed unit 20 and dispensing it in a main vial 8,
- a gauge 5 for measuring the radioactive activity present in the main vial 8,
- means 12 for diluting the radioactivity with a physiological saline to obtain a predetermined concentration of activity in the liquid in the main vial 8,
- transfer means 4 for transferring a predetermined volume of radioactive liquid through pipes 21 from the main vial 8 to at least one vial 3,
- electronic scales 22 for weighing the vial 3 containing the liquid,
- a programmable control unit 13 for controlling at least the transfer means 4 and the transfer means 12, based on instruction signals received by the unit 13 and
- a remote data processing unit 17, interfaced with the programmable control unit 13 to send the control unit instruction signals processed according to at least one parameter (for example, radioactive activity, volume, time) relating to one or more doses to be dispensed.

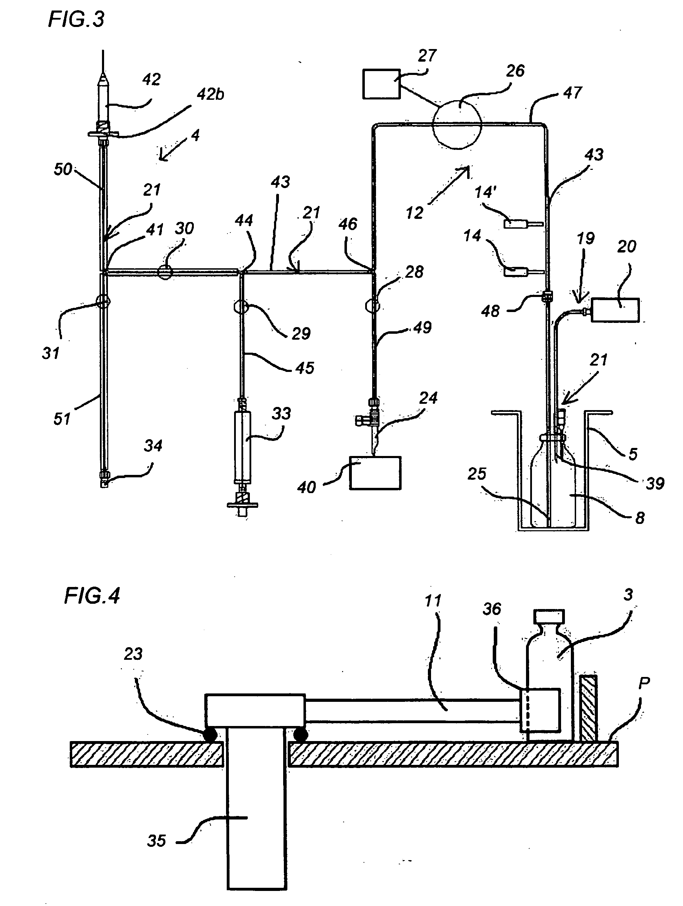

With reference to Figure 3, the means 19 for drawing out the radioactive liquid advantageously comprise a first end connected to the feed unit 20 and a second end equipped with an injector 39 positioned inside the main vial 8.

Again with reference to Figure 3, the transfer means 4 and 12 also comprise a peristaltic pump 26 controlled by an encoder 27.

The pump 26 is removably applied on the pipes 21 in a position between the vial 3 and the main vial 8.

This type of pump 26 can be controlled by the encoder 27 in such a way that the liquid flow rate is adapted according to the vial 3 filling step.

In particular, the encoder 27 keeps the pump 26 at a speed close to the maximum value up to a predetermined volume "V0" close to the end of the vial 3 filling operation, then slows to a lower speed which allows a high degree of precision at the end of dispensing.

Moreover, advantageously, the transfer means 4 may comprise at least one sensor 14/14' for checking for the presence of any air in the pipes 21.

If the sensor 14/14' detects the presence of air bubbles, it allows a check to ensure that the volume of liquid processed by the pump 26 is effectively that poured into the vial 3, or correction of the dispensing to reach the desired volume.

In particular with reference to Figure 3, the pipes 21 of the transfer means 4 comprise:
- a dispenser 42 for injecting the liquid into the vial 3, positioned at the end of a first pipe 50 connected to a first union tee 41,
- a first controlled valve 30 fitted between the union tee 41 and a second union tee 44, for the automatic integrity test of the filter 42b (an integral part of the dispenser 42),
- a second pipe 43 positioned between the second union tee 44 and a third union tee 46,
- a third pipe 45 positioned between the second union tee 44 and a storage unit 33;
- a second controlled valve 29 fitted between the second union tee 44 and the storage unit 33;
- one or more bubble sensors 14/14' fitted between the pump 26 and the end 25 of the fifth pipe 47;
- a fourth pipe 49 positioned between the third union tee 46 and an end 24 for drawing from a physiological saline feed unit 40;
- a third controlled valve 28 fitted between the third union tee 46 and the physiological saline unit 40;
- a fifth pipe 47 positioned between the third union tee 46 and an end 25 for drawing from the main vial 8 and which passes through the pump 26 in an intermediate position;
- a union 48, if present, positioned between the sensor 14/14' and the end 25;
- a sixth pipe 51 positioned between the first union tee 41 and an end 34 for connecting a compressed air supply, needed for connection of the system for measuring filter 42b integrity;
- a fourth controlled valve 31 fitted between the first union tee 41 and the end 34.

During machine operation, the end 34 is used to test correct operation of the outfeed filter 42b close to the dispenser 42.

The particular feature of the filter is that it allows air to pass through it only if liquids did not previously pass through it, and once wet it breaks if air passes through it at a pressure which is above a predetermined threshold, for example 3 bar.

The filter test consists of closing the valve 30 and opening the valve 31 and pressurising the filter with compressed air entering from the end 34 until the filter breaks. It must break when an air pressure of 3 bar is exceeded. This validates the entire production.

If the filter does not break under these conditions, production was performed using a broken filter and therefore must be invalidated.

The peak pressure in the hydraulic pipe is measured by a commercial device not connected to the crimper, however, the control software "asks" the operator if this test was passed or failed, recording the result in the production report in a definitive, non-modifiable way.

Advantageously, in the embodiment described the diluting means 12 share with the transfer means the pipe 49 connected to the physiological saline feed unit 40 and the valve 28 which operates on the pipe 49 in a position between the first end 24 and the pump 26, with the aim of preventing the drawing of physiological saline from the feed unit 40.

With reference to Figure 2, the programmable control unit 13 is preferably a PLC (Programmable Logic Controller) unit, that is to say, a commercial electronic component, which may be dedicated to automated control of the fractionating process.

As its main function the unit 13 runs a program and processes the digital and analogue signals arriving from sensors and actuators present in the industrial system.

Advantageously, the programmable control unit 13 also controls the sensors 14/14', a bar code reader 37 and one or more relays 38, which form an integral part of the PLC and are operated by commands issued to the PLC programming unit.

A remote data processing unit 17 interfaces with the programmable control unit 13. The former is connected to a plurality of peripherals, which may be any of the following: modem, CD/DVD writer, keyboard, USB key, labeller, monitor, printer or the like.

With reference to Figure 4, the fractionating machine disclosed also comprises a mechanism 11 for moving the vial 3 on the machine 1 work surface "P".

The rotary arm preferably projects over the supporting surface "P" and also comprises a seal 23 positioned on the hub 35 to protect the mechanism 11 from external gasses and liquids, normally used to clean the machine 1.

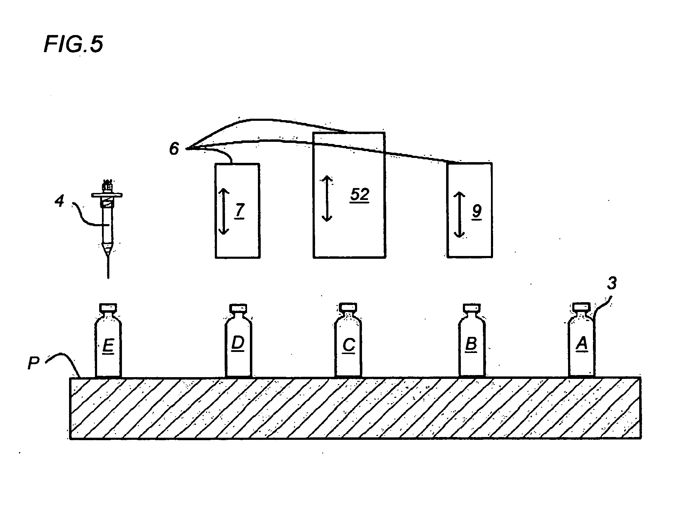

With reference to Figure 5 the vial 3 is moved between a first vial 3 pick up operating position "A" and a filling position "E".

The first vial 3 pick up operating position "A" is followed by a second position "B" for removal/placing of an aluminium cap for hermetically sealing the vial 3.

The second position "B" is followed by a third position "C", consisting of a crimper gripper 52 needed, after filling and repositioning of the rubber stopper (see next point) and the aluminium cap, for vial crimping (folding over of the aluminium cap on the vial neck by mechanical deformation).

The position "C" precedes a fourth position "D" for removal/insertion of a rubber stopper on the vial 3.

The position "D" is followed by the position "E" already referred to, for filling the vial 3 with the radioactive liquid.

Advantageously, positions "B", "C" and "D" are covered by a crimping unit 6 which allows the vial 3 to be opened without making a hole in the rubber stopper.

Respectively at the operating positions described above, in particular position "B" corresponds to a device for removal/insertion 9 of the aluminium cap, position "C" corresponds to the crimping station 52 for the filled vial and position "D" corresponds to a device for removal/insertion 7 of the rubber stopper on the vial 3.

With reference to Figure 2, the machine disclosed has a handling section M which carries out the fractionating process according to the control unit 13 programming, and which interfaces with a supervision section S, equipped with the processing unit 17.

As already indicated, the process unit 13 and the programming/supervision unit 17 interface by means of the interfaces 16.

The supervision section S also interfaces with the gauge 5, which may be outside the machine 1.

During operation, a fractionating machine in accordance with the present invention has a first step of programming the fractionating process carried out by the unit 17 and which ends with the preparation of a first set of operating instructions and if necessary with the printing of labels on the vials 3 to be delivered.

During the next step, the unit 13 carries out the fractionating process, checking the machine 1 operations and in particular:
- checks the integrity of the pipes by performing an air bubble presence test using the sensors 14/14';
- runs an automatic calibration process for the "pump - pipes in use" system, using the sensors 14/14', optimising performance precision;
- if the test is positive, checks the arrival of the radioactive activity required in the main vial 8 and if necessary achievement of the dilution necessary according to the instructions received. If the incoming radioactivity does not match that required, it sends a correction signal to the unit 17 which if necessary modifies the set of operating instructions and prints the label for one or more of the vials 3 again. During this step, the storage unit 33 is used to transfer volumes of liquid from the main vial, in order to calculate the initial concentration of the radioactive liquid. In more detail, the calculation of the concentration consists in moving known volumes of radioactive liquid from the main vial to the storage unit 33 using the pump and measuring a variation in the radioactivity in the vial. This makes it possible (for example by interpolating various points of a straight line) to calculate the concentration and, inverting the formula, work back to the volume.
   At this point, if the initial volume is known, it is possible to dilute the liquid with a known volume to achieve the concentration required by the customer;
- activate vial 3 handling towards and from the dispensing position E described above;
- start the process of dispensing the radioactive liquid into the individual vials 3 by controlling the volume transferred by the pump 26 and if necessary the weight measured by the scales 22. During the dispensing process, the unit 13 modifies the dispensing speed/time according to the filling step, possible air bubbles detected by the sensors 14/14' and the weight of the liquid dispensed, effectively measured in line by the scales 22;
- at the end of dispensing according to the instructions received, the unit 13 may empty the pipes and wash them with physiological saline, draining the residual liquid into dedicated vials labelled as "purge", which are closed, crimped and moved away from the dispensing zone like the other vials produced. At the end of the washing, the machine may be used by the operator to substitute the pipes or update the equipment.

Advantageously, all of the steps just described are carried out without direct contact between the operator and the pipes full of radioactive liquid, thus reducing the chance of operator exposure, even when the fractionating process has to be reprogrammed, and if necessary the labels to be applied to the vials for delivery have to be reprinted.

The invention described above is susceptible of industrial application and may be modified and adapted in several ways without thereby departing from the scope of the inventive concept as claimed.

## Claims

1. An automatic machine (1) for dispensing measured doses of a radioactive liquid in one or more vials (3) for delivery comprising:
- means (19) for drawing the radioactive liquid from a feed unit (20) until a predetermined radioactive activity is reached, and dispensing the liquid drawn into a main vial (8),
- a gauge (5) for measuring the radioactive activity value present in the main vial (8),
- means (12) for diluting the radioactivity with a physiological saline to obtain a predetermined concentration of radioactivity in the liquid in the main vial (8),
- transfer means (4) for transferring a predetermined volume of radioactive liquid through pipes (21) from the main vial (8) to at least one vial (3),
- electronic scales (22) for weighing the vial (3) containing the liquid,
- a programmable control unit (13) for controlling at least the transfer means (4), based on instruction signals received by the unit (13);
- a remote data processing unit (17), interfaced with the programmable control unit (13) to send the control unit (13) instruction signals processed according to at least one parameter relating to one or more doses to be dispensed.

2. The machine according to claim 1, wherein the means (19) for drawing out the radioactive liquid comprise a first end connected to the feed unit (20) and a second end (39) positioned inside the main vial (8).

3. The machine according to either of the foregoing claims, wherein the diluting means (12) comprise pipes (43) having a first end (24) connected to a physiological saline feed unit (40) and a second end (25) positioned inside the main vial (8).

4. The machine according to any of the foregoing claims, wherein the transfer means (4) also comprise a peristaltic pump (26) controlled by an encoder (27), it being possible to removably apply the pump (26) on the pipes (21) in a position between the vial (3) for delivery and the main vial (8).

5. The machine according to any of the foregoing claims, wherein the transfer means (4) also comprise at least one sensor (14/14') for checking for the presence of any air in the pipes (21).

6. The machine according to any of the foregoing claims from claim 3, wherein the diluting means (12) also comprise a valve (28) removably applied on the pipes (43) in a position fitted between the first end (24) and the pump (26) to prevent the drawing of physiological saline from the feed unit (40).

7. The machine according to any of the foregoing claims, wherein the transfer means (4) also comprise a valve (30) fitted between an intersection (41) and the dispenser (42) in order to shut off supply to the latter.

8. The machine according to any of the foregoing claims, wherein the programmable control unit (13) controls at least one bubble sensor (14/14'), a bar code reader (37) and one or more relays (38).

9. The machine according to any of the foregoing claims, also comprising a mechanism (11) for moving the vial (3) on the machine (1) work surface (P) between a first vial (3) pick up operating position (A) and a filling position (E).

10. The machine according to claim 9, wherein the mechanism (11) comprises a rotary arm projecting over the supporting surface (P).

11. The machine according to claim 9, wherein the first vial (3) pick up operating position (A) is followed by a second position (B) for removal/insertion of an aluminium cap for hermetically sealing the vial (3), the second position (B) is followed by a third position (D) for removal/insertion of a rubber stopper on the vial (3), the third position (D) is followed by the position (E) for filling the vial (3) with the liquid, the filling position (E) is followed by a fifth position (C) for crimping the filled vial (folding over of the aluminium cap on the vial neck by mechanical deformation) to hermetically seal the vial (3), after repositioning the rubber stopper and the aluminium cap.

12. The machine according to claim 4, wherein the encoder (27) controls the pump (26) in such a way as to obtain operation at the maximum speed until a predetermined volume (V) is required to fill the vial, in order to improve pump flow rate precision.

13. A method for fractionating into one or more doses an incoming radioactive activity, supplied by a radioactive liquid drawn from a feed unit (20), and fed to vials (3) for delivery, comprising the following steps:
- preparing a processing unit (17) for processing a first set of operating instructions according to an expected radioactivity of the dose contained in the vial (3) at a predetermined date,
- preparing a programmable unit (13) to perform the process of fractionating the radioactivity into doses having predetermined characteristic parameters in response to the set of instructions received from the processing unit (17),
- checking at least one characteristic parameter of the radioactive activity expected to arrive in the main vial (8) and, if said parameter does not match a predetermined value, sending a correction signal to the unit (17) to modify the set of operating instructions,
- dispensing of the radioactivity into the individual vials (3) programmed according to the current operating instructions, by controlling the volume transferred and/or the weight of the liquid dispensed in the vial (3) wherein the unit (13) carries out an air bubble presence test in the fractionated liquid and if necessary sends a correction signal to the unit (17).

14. The method according to claim 13, wherein the unit (13) carries out a step of emptying and washing with physiological saline.

## Patentansprüche

1. Automatische Maschine (1) zum Abgeben abgemessener Dosen einer radioaktiven Flüssigkeit in eine oder mehrere Phiolen (3) zur Auslieferung, umfassend:
- Mittel (19) zum Abziehen der radioaktiven Flüssigkeit aus einer Zufuhreinheit (20), bis eine vorbestimmte radioaktive Aktivität erreicht ist, und Abgeben der abgezogenen Flüssigkeit in eine Hauptphiole (8),
- eine Messeinrichtung (5) zum Messen des radioaktiven Aktivitätswertes, der in der Hauptphiole (8) vorhanden ist,
- Mittel (12) zum Verdünnen der Radioaktivität mit einer physiologischen Salzlösung, um eine vorbestimmte Radioaktivitätskonzentration in der Flüssigkeit in der Hauptphiole (8) zu erhalten,
- Übertragungsmittel (4) zum Übertragen eines vorbestimmten Volumens an radioaktiver Flüssigkeit durch Rohre (21) von der Hauptphiole (8) zu mindestens einer Phiole (3),
- eine elektronische Waage (22) zum Wiegen der die Flüssigkeit enthaltenden Phiole (3),
- eine programmierbare Steuereinheit (13) zum Steuern zumindest der Übertragungsmittel (4) auf der Basis von Anweisungssignalen, die von der Einheit (13) empfangen werden;
- eine entfernte Datenverarbeitungseinheit (17), die mit der programmierbaren Steuereinheit (13) über eine Schnittstelle verbunden ist, um der Steuereinheit (13) Anweisungssignale zu senden, die gemäß mindestens einem Parameter verarbeitet sind, der mit einer oder mehreren abzugebenden Dosen in Beziehung steht.

2. Maschine nach Anspruch 1, wobei die Mittel (19) zum Abziehen der radioaktiven Flüssigkeit ein erstes Ende, das mit der Zufuhreinheit (20) verbunden ist, und ein zweites Ende (39), das innerhalb der Hauptphiole (8) angeordnet ist, umfassen.

3. Maschine nach einem der vorhergehenden Ansprüche, wobei die Verdünnungsmittel (12) Rohre (43) mit einem ersten Ende (24), das mit einer Zufuhreinheit (40) für physiologische Salzlösung verbunden ist, und einem zweiten Ende (25), das innerhalb der Hauptphiole (8) angeordnet ist, umfassen.

4. Maschine nach einem der vorhergehenden Ansprüche, wobei die Übertragungsmittel (4) auch eine Schlauchquetschpumpe (26) umfassen, die von einem Encoder (27) gesteuert ist, wobei es möglich ist, die Pumpe (26) abnehmbar an den Rohren (21) in einer Position zwischen der Phiole (3) zur Auslieferung und der Hauptphiole (8) anzubringen.

5. Maschine nach einem der vorhergehenden Ansprüche, wobei die Übertragungsmittel (4) auch mindestens einen Sensor (14/14') umfassen, um auf das Vorhandensein von irgendwelcher Luft in den Rohren (21) zu prüfen.

6. Maschine nach einem der vorhergehenden Ansprüche von Anspruch 3, wobei die Verdünnungsmittel (12) auch ein Ventil (28) umfassen, das abnehmbar an den Rohren (43) in einer Position angebracht ist, die zwischen dem ersten Ende (24) und der Pumpe (26) eingerichtet ist, um das Abziehen von physiologischer Salzlösung aus der Zufuhreinheit (40) zu verhindern.

7. Maschine nach einem der vorhergehenden Ansprüche, wobei die Übertragungsmittel (4) auch ein Ventil (30) umfassen, das zwischen einem Zwischenabschnitt (41) und der Abgabevorrichtung (42) eingerichtet ist, um die Zufuhr zu der letzteren zu unterbrechen.

8. Maschine nach einem der vorhergehenden Ansprüche, wobei die programmierbare Steuereinheit (13) zumindest einen Blasensensor (14/14'), einen Strichcodeleser (37) und ein oder mehrere Relais (38) steuert.

9. Maschine nach einem der vorhergehenden Ansprüche, die auch einen Mechanismus (11) zum Entnehmen der Phiole (3) auf der Arbeitsfläche (P) an der Maschine (1) zwischen einer ersten Aufnahmebetriebsposition (A) und einer Füllposition (E) der Phiole (3) umfasst.

10. Maschine nach Anspruch 9, wobei der Mechanismus (11) einen Dreharm umfasst, der über die Trägerfläche (P) vorsteht.

11. Maschine nach Anspruch 9, wobei der ersten Aufnahmebetriebsposition (A) der Phiole (3) eine zweite Position (B) zum Entfernen/Einsetzen einer Aluminiumkappe zum hermetischen Abdichten der Phiole (3) folgt, der zweiten Position (B) eine dritte Position (D) zum Entfernen/Einsetzen eines Gummistopfens auf der Phiole (3) folgt, der dritten Position (D) die Position (E) zum Füllen der Phiole (3) mit der Flüssigkeit folgt, der Füllposition (E) eine fünfte Position (C) zum Krimpen der gefüllten Phiole (Umschlagen der Aluminiumkappe auf den Phiolenhals durch mechanische Verformung) folgt, um die Phiole (3) hermetisch abzudichten, nachdem der Gummistopfen und die Aluminiumkappe umpositioniert worden sind.

12. Maschine nach Anspruch 4, wobei der Encoder (27) die Pumpe (26) auf eine solche Weise steuert, dass ein Betrieb mit der maximalen Geschwindigkeit erhalten wird, bis ein vorbestimmtes Volumen (V) erforderlich ist, um die Phiole zu füllen, um die Genauigkeit der Pumpendurchflußmenge zu verbessern.

13. Verfahren zum Fraktionieren einer hereinkommenden radioaktiven Aktivität in eine oder mehrere Dosen, welche durch eine radioaktive Flüssigkeit zugeführt wird, die von einer Zufuhreinheit (20) abgezogen wird, und Phiolen (3) zur Auslieferung zugeführt wird, mit den folgenden Schritten, dass:
- eine Verarbeitungseinheit (17) zum Verarbeiten eines ersten Satzes von Betriebsanweisungen gemäß einer erwarteten Radioaktivität der in der Phiole (3) an einem vorbestimmten Datum enthaltenen Dosis vorbereitet wird,
- eine programmierbare Einheit (13) vorbereitet wird, um den Prozess des Fraktionierens der Radioaktivität in Dosen, die vorbestimmte charakteristische Parameter aufweisen, in Ansprechen auf den Satz von Anweisungen, die von der Verarbeitungseinheit (17) empfangen werden, durchzuführen,
- zumindest ein charakteristischer Parameter der radioaktiven Aktivität geprüft wird, von der erwartet wird, dass sie in der Hauptphiole (8) ankommt, und wenn der Parameter nicht zu dem vorbestimmten Wert passt, ein Korrektursignal zu der Einheit (17) gesendet wird, um den Satz von Betriebsanweisungen abzuwandeln,
- die Radioaktivität in die einzelnen Phiolen (3) programmiert gemäß den gegenwärtigen Betriebsanweisungen abgegeben wird, indem das übertragene Volumen und/oder das Gewicht der in die Phiole (3) abgegebenen Flüssigkeit gesteuert wird, wobei die Einheit (13) einen Luftblasenanwesenheitstest in der fraktionierten Flüssigkeit ausführt und ggf. ein Korrektursignal an die Einheit (17) sendet.

14. Verfahren nach Anspruch 13, wobei die Einheit (13) einen Schritt eines Entleerens und Waschens mit physiologischer Salzlösung ausführt.

## Revendications

1. Machine automatique (1) pour distribuer des doses mesurées d'un liquide radioactif dans un ou plusieurs flacons (3) pour la fourniture, comprenant :
- des moyens (19) pour tirer le liquide radioactif à partir d'une unité d'alimentation (20) jusqu'à ce qu'une activité radioactive prédéterminé soit atteinte et distribuer le liquide prélevé dans un flacon principal (8),
- un instrument de mesure (5) pour mesurer la valeur d'activité radioactive présente dans le flacon principal (8),
- des moyens (12) pour diluer la radioactivité avec un sérum physiologique pour obtenir une concentration de radioactivité prédéterminée dans le liquide dans le flacon principal (8),
- des moyens de transfert (4) pour transférer un volume prédéterminé de liquide radioactif à travers des tubes (21) du flacon principal (8) à au moins un flacon (3),
- des bascules électroniques (22) pour peser le flacon (3) contenant le liquide,
- une unité de commande programmable (13) pour commander au moins les moyens de transfert (4), sur la base de signaux d'instruction reçus par l'unité (13) ;
- une unité de traitement de données à distance (17) interfacée avec l'unité de commande programmable (13) pour transmettre à l'unité de commande (13) des signaux d'instruction élaborés en fonction d'au moins un paramètre relatif à une ou plusieurs doses à distribuer.

2. Machine selon la revendication 1, dans laquelle les moyens (19) pour tirer le liquide radioactif comprennent une première extrémité connectée à l'unité d'alimentation (20) et une deuxième extrémité (39) positionnée à l'intérieur du flacon principal (8).

3. Machine selon l'une quelconque des revendications précédentes, dans laquelle les moyens de dilution (12) comprennent des tubes (43) ayant une première extrémité (24) connectée à l'unité d'alimentation de sérum physiologique (40) et une deuxième extrémité (25) positionnée à l'intérieur du flacon principal (8).

4. Machine selon l'une quelconque des revendications précédentes, dans laquelle les moyens de transfert (4) comprennent également une pompe péristaltique (26) contrôlée par un encodeur (27), avec la possibilité d'appliquer la pompe (26) de manière amovible sur les tubes (21) dans une position entre le flacon (3) pour la distribution et le flacon principal (8).

5. Machine selon l'une quelconque des revendications précédentes, dans laquelle les moyens de transfert (4) comprennent également au moins un capteur (14/14') pour contrôler la présence d'air dans les tubes (21).

6. Machine selon l'une quelconque des revendications précédentes à partir de la revendication 3, dans laquelle les moyens de dilution (12) comprennent également une vanne (28) appliquée de manière amovible sur les tubes (43) dans une position entre la première extrémité (24) et la pompe (26) pour empêcher le prélèvement de sérum physiologique à partir de l'unité d'alimentation (40).

7. Machine selon l'une quelconque des revendications précédentes, dans laquelle les moyens de transfert (4) comprennent également une vanne (30) installée entre une intersection (41) et le distributeur (42) de manière à couper l'alimentation de ce dernier.

8. Machine selon l'une quelconque des revendications précédentes, dans laquelle l'unité de commande électronique (13) commande au moins un détecteur de bulles (14/14'), un lecteur de code à barres (37) et un ou plusieurs relais (38).

9. Machine selon l'une quelconque des revendications précédentes, comprenant également un mécanisme (11) pour déplacer le flacon (3) sur la surface de travail (P) de la machine (1) entre une première position (A) fonctionnelle de prélèvement du flacon (3) et une position de remplissage (E).

10. Machine selon la revendication 9, dans laquelle le mécanisme (11) comprend un bras rotatif faisant saillie au-dessus de la surface de support (P).

11. Machine selon la revendication 9, dans laquelle première position (A) fonctionnelle de prélèvement du flacon (3) est suivie par une deuxième position (B) pour l'enlèvement/insertion d'un bouchon en aluminium pour sceller hermétiquement le flacon (3), la deuxième position (B) est suivie par une troisième position (D) pour l'enlèvement/insertion d'un élément d'arrêt en caoutchouc sur le flacon (3), la troisième position (D) est suivie par la position de remplissage (E) pour remplir le flacon (3) avec le liquide, la position de remplissage (E) est suivie par une cinquième position (C) pour sertir le flacon rempli (pliage du bouchon d'aluminium sur le goulot du flacon par déformation mécanique) pour sceller hermétiquement le flacon (3), après repositionnement de l'élément d'arrêt en caoutchouc et du bouchon en aluminium.

12. Machine selon la revendication 4, dans laquelle l'encodeur (27) commande la pompe (26) de manière à obtenir un fonctionnement à la vitesse maximale jusqu'à un volume prédéterminé (V) soit requis pour remplir le flacon, de manière à améliorer la précision de débit de la pompe.

13. Procédé pour fractionner en une ou plusieurs doses une activité radioactive reçue, délivré par un liquide radioactif prélevé à partir d'une unité d'alimentation (20) et alimenté dans des flacons (3) pour la fourniture, comprenant les étapes suivantes :
- la préparation d'une unité de traitement (17) pour traiter un premier ensemble d'instructions de fonctionnement en fonction d'une radioactivité supposée de la dose contenue dans le flacon (3) à une date prédéterminée,
- la préparation d'une unité programmable (13) pour effectuer le processus de fractionnement de la radioactivité en doses ayant des paramètres caractéristiques prédéterminés en réponse à l'ensemble d'instructions reçues de l'unité de traitement (17),
- le contrôle d'au moins un paramètre caractéristique de l'activité radioactive supposée se produire dans le flacon principal (8) et, si ledit paramètre ne correspond pas à une valeur prédéterminée, envoyer un signal de correction à l'unité (17) pour modifier l'ensemble d'instructions de fonctionnement,
- distribuer la radioactivité dans les flacons individuels (3) programmés en fonction des instructions de fonctionnement actuelles, en contrôlant le volume transféré et/ou le poids du liquide distribué dans le flacon (3), dans lequel l'unité (13) exécute un test de présence de bulles d'air dans le liquide fractionné et, si nécessaire, envoie un signal de correction à l'unité (17).

14. Procédé selon la revendication 13, dans lequel l'unité (13) exécute une étape de vidage et lavage avec du sérum physiologique.
